# EUROPEAN PATENT APPLICATION

(11) **EP 0 937 447 A2**
(43) Date of publication of application: **25.08.1999**
(21) Application number: 99103150.1
(22) Date of filing: 18.02.1999
(51) Int. Cl.: A61F 15/00

(54) **Protective clothing for medical use**

(30) Priority: 20.02.1998 IT RE980020; 15.02.1999 IT RE990021
(71) Applicant: Catellani, Nello, Canossa (Reggio Emilia) (IT)
(72) Inventor: Catellani, Nello, Canossa (Reggio Emilia) (IT)
(74) Representative: Lecce, Giovanni

(57) **Abstract**

Clothes from elastic material, flexible and insulating, with sealing strips (2) and/or tubular elements (20) are utilised to cover body parts ill or infected or the like, which must be temporary protected and/or insulated from the adjoining environment areas or vice-versa, during medical or paramedical treatments, independent or collective, such as those of rehabilitation or the like, to which the treated patients must be subjected.

## Description

The present invention relates to a line of sealing clothes for medical or paramedical use, wherein all the clothes are substantially realised from flexible and insulating elastic materials, such as for instance natural or synthetic latex, PVC or the like, and are so configured to cover parts of the human body affected by infections, psoriasis, decubitus ulcers and the like, or subjected to various problems, such as for instance incontinence and the like, to protect them and to create an insulation between them and the external environment or vice-versa, during the periods wherein the concerned patients must be subjected to particular care and/or rehabilitation practices. All the clothes are provided with individual or multiple sealing strips, or sealing inflatable tubular elements, which develop along the peripheral edges of the openings through which the clothes are put on.

As is known, the patients who have undergone severe traumas with exposed wounds or who have contracted skin infections, such as psoriasis or the like, or who are affected by decubitus ulcers, or who, being late in life or due to particular insufficiencies, suffer from incontinence, or who for still other reasons, must necessarily be treated with intensive recovering treatments, so that their physical conditions may be brought back to a gradual general rehabilitation and a restoring of their normal body functions, or, to the extent possible, or treated with a stimulation of their functions so that they may somehow tend towards such recoveries to proceed then to their maintaining. As is also known, in the application of the necessary curative treatments and/or in carrying on the rehabilitation practices foreseen, very often conditions create that are unacceptable both from the human point of view and especially from the health-sanitary point of view. For example, it is evident that, during the rehabilitation treatments including water-gymnastic exercises in swimming pools, it is absolutely unthinkable to put together patients suffering from incontinence and patients who do not suffer at all from this disorder, as there exists the risk that somebody affected from this disorder unconsciously releases his or her faeces and/or urine, so that the water wherein also other people are carrying on their gymnastic exercises, becomes abruptly polluted, unhealthy and hygienically dangerous. In other conditions, related to the presence of patients affected by infections, ulcers and the like, there are the same problems in case of rehabilitation practices in swimming pools, in case of contacts with other patients or in case of common use of objects that may act as infection carriers, and it may also happen that some patients cannot be submitted to particular treatments because of the presence on their body of localised infections or the like.

Object of the present invention is to obviate the aforesaid drawbacks. The invention, as is characterised by the claims, solves the problem by means of a line of protective sealing clothes, for medical or paramedical use, through which the following results are achieved: the clothes are made from elastic, flexible and insulating material having a very reduced thickness, to render their use easy and quick; they may be of the re-usable type or of the disposable type; the complete line comprises clothes suitable for all the particular needs and for all the body parts, independent, extended and/or combined to each other; all the clothes comprise, in correspondence of the openings through which they are put on, individual or multiple strips; alternatively or in combination, the outermost strip, which may be a single and independent strip or also associated to one or more elastic strips, is made up by a tubular body, elastic and inflatable, combined with a positioning surface belt.

The advantages achieved by the present invention essentially consist in that such clothes allow to temporary cover, protect and insulate any body parts from the adjoining ones, or vice-versa, and to allow consequently the unconditional utilisation of any type of therapy, medical or paramedical, suitable and necessary to the patients for possible curative and/or rehabilitation treatments.

The invention will be now described in detail, according to some embodiment solely reported by way of non limiting example, with reference to the attached drawings, wherein:
Figures 1-4 show elements of the line of protective clothes, specifically designed for the protection of the lower limbs or parts thereof,
Figure 5 and 6 show elements of the line of protective clothes, specifically designed for the protection of the legs and the pelvis;
Figure 7 shows an element of the line of protective clothes, specifically designed for the protection of the bust or parts thereof,
Figure 8 shows an element of the line of protective clothes, specifically designed for the protection of the shoulders;

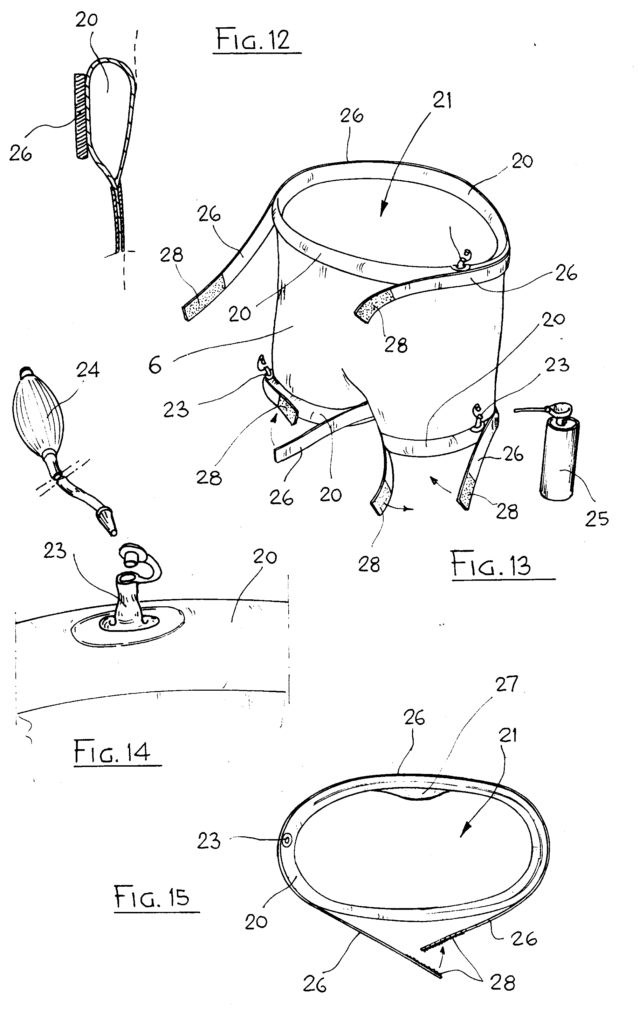

Figures 9-11 show elements of the line of protective clothes, specifically designed for the protection of the forelimbs or parts thereof;
Figure 12 shows the cross-section of a sealing annular element for protective clothes:
Figure 13 shows a perspective overall view of a cloth provided with the sealing annular element;
Figure 14 shows an example of inflation and sealing valve of a tubular body constituting the sealing annular element, and
Figure 15 shows the cross-section of a particular configuration of the sealing device for a specific application.

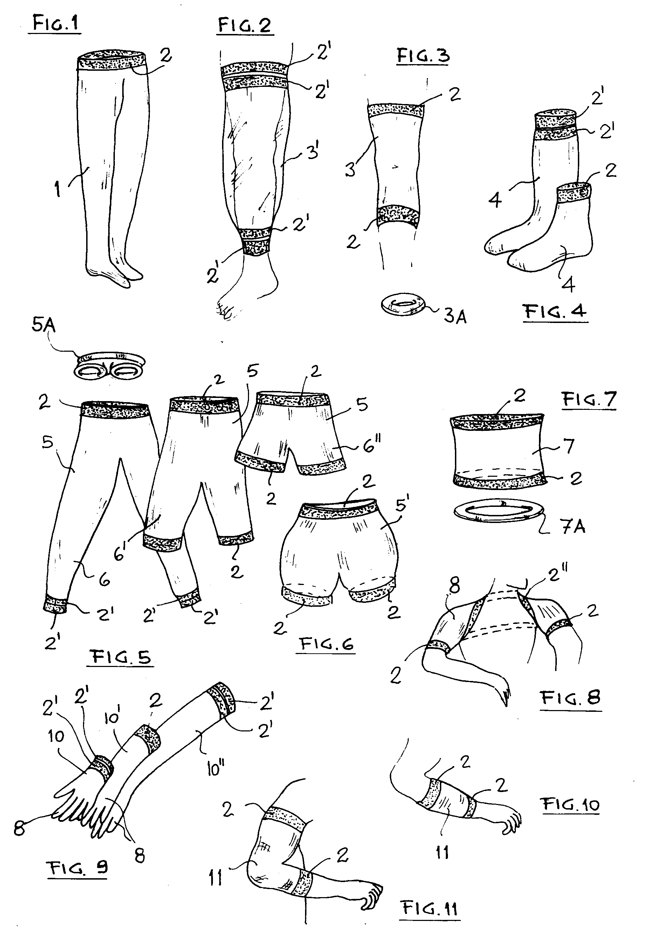

The figures show a line of protective sealing clothes for medical or paramedical use, wherein the clothes are realised from elastic, flexible and insulating material, such as for instance natural or synthetic latex or the like, of a vary reduced thickness. Among the various possible forms of clothes suitable for the protection of well defined parts of the human body, pantyhose (1) forms are realisable, suitable to cover, in one only piece without joints, the feet, the complete legs and the pelvis up to the waist; around the waist at least a sealing peripheral strip (2) is located which prevents any infiltration and/or contact of the covered body part with the external environment, and vice-versa. The sealing strip may be realised from at least a strip (2) or several adjoining annular strips (2') from a same elastic material, preferably with a reinforced thickness, possibly treated or impregnated with soil, non-irritating surface means facing the inside, which closely adhere to the skin during the sealing stages. For the partial protection of the legs, leggings (3), (3') have been realised, more or less extended according to the covering requirements, also provided with sealing strips (2), (2') at the open ends for the passage of the limbs. In the same way, for the protection of the feet, socks (4), (4') have been realised having long or short legs, always provided with sealing strips (2), (2) at the upper fitting ends. For the protection of the pelvis, trousers or pants (5), (5') with long (6), medium (6') or short (6") legs have been realised, complete with sealing strips (2), (2') at the waist and along the legs. The protection of the bust or part thereof is provided by clothes (7) having a form substantially similar to that of the conventional body-belt or part thereof complete with sealing strips (2), (2') at the upper and lower fitting ends. The protection of the shoulders and part of the back and/or the chest is provided by an enveloping cloth (8) which is put on through the arms and which also comprises adequate peripheral sealing edges (2), (2'); the parts covering the shoulders may be developed also along the arms, up to the hands, possibly comprised. Gloves (9) with armlets of various length, wrist- (10), elbow- (10") and armpit- (10") long, are designed for the protection of the forelimbs, in the same way as the armlets (11) designed for the protection of the elbow, arm or forearm areas. All the clothes may be so configured as to adhere to the body parts, or they may be ample, as in the cases indicated by (3') and (5') in Figures 2 and 6, not to be adherent, in which case the sealing is all the same maintained through the special strips (2), (2').

In Figures 3-5 and 7, the illustrated clothes are also represented according to a preferred, non limiting, tailoring solution, i.e. ring-like rolled up around openings (3A), (5A), (7A), so that the clothes may be put on more easily and quickly. It is however obvious that other tailoring forms of a conventional type may be adopted without departing from the invention scope.

In a different, non limiting, solution, in fact, the clothes may be realised from a different elastic, flexible and insulating material of very reduced thickness, such as for instance PVC, covered outside and possibly also inside, with acrylic fabric or a like fabric, to render it soil and pleasant on the touch with the skin.

All the annular openings (21) through which the clothes can be put on may be provided with, instead of the sealing strips (2), (2') or in association with them, with a tubular border (20) with at least a valve. By means of any hand-pump (24) or compressed air cylinder (25) or like means, air is let through valve (23) into the tubular border (20) so as to inflate it and to create a sealing edge which fits to the body part on which the cloth has been put on, without blocking and/or contrasting blood circulation. The tubular element (20) may be realised separately and be thereafter connected to the annular opening (21) by means of vulcanisation on the cloth, or it may be directly obtained by folding on itself the border of each opening, with vulcanisation of the cloth of which it is a part.

The inflated tubular element (20) is maintained against the body of the user by an external non elastic belt (26) that fits to the skin surface. The belt is closed by buckles or like means, such as for instance hooks, press-studs, pressure sensible adhesives (28) of the Velcro type, or the like. The external belt (26) makes the positioning easier as it forms, being not elastic, a ring non-extensible towards the outside, which causes the tubular body to expand, during the inflation, only towards the inside and in perfect adherence to the skin, following it possible curves.

The valve (23) may be a plug-in valve, of the type usually adopted for children's small life-rings and/or dinghies, so as not to constitute surface disturbances that can get in touch with the skin; they may, or may not, be provided with nonreturn internal membranes.

The sealing clothes of the type like pants, trousers, body-belts and the like may be provided with tubular elements (20) that comprise a back pocket (27) filled with silicone or other fluid plastic products, facing towards the inside and placed in alignment with the back-zone corresponding to the spine which, as is known, has a natural hollow. In this way, having put on the cloth, tightened the belt (26) and inflated the tubular element (20), the silicone or the like comprised in the pocket (27) is pressed against said zone, fitting in the hollow and forming a sealing on the same.

While the present invention has been described according to a qualitative line of clothes, solely reported by way of non limiting example, it will be obvious to those skilled in the art that various changes in the structures, forms, details, extensions and combination of the parts may be introduced without departing from its scope.

## Claims

1. A line of sealing protective clothes for medical or paramedical use, characterised in that it comprises clothes of various types and shapes, specifically corresponding to one or more adjoining parts of the human body, made from elastic, flexible and insulating material, and provided with sealing means constituted by annular strips (2), (2') and/or inflatable elastic tubular elements (20), said sealing means being located in correspondence of the openings (21) through which said clothes are put on.

2. The line of sealing protective clothes according to claim 1, characterised in that the elastic, flexible and insulating material adopted for the realisation of the clothes is natural or synthetic latex, PVC or the like, having a very reduced thickness.

3. The line of sealing protective clothes according to claims 1 and 2, characterised in that the sealing annular strips (2), (2') located in correspondence of the openings (21) through which said clothes are put on are made from the same elastic, flexible and insulating material of the clothes, have a preferably reinforced thickness, are in number of one or two adjoining strips, and may or may not be provided and possibly treated or engaged with soil non- irritating surface means facing the inside, which are caused to closely adhere to the skin during the sealing steps.

4. The line of sealing protective clothes according to claims 1 and 2, characterised in that the inflatable elastic tubular elements (20) are realised separately and connected to the annular opening (21) by vulcanisation on the cloth, or directly obtained by folding on itself the borders of every opening, with direct vulcanisation on the clothes of which they make part; said clothes being covered outside and possibly inside with acrylic fabric or the like, soil and pleasant on the touch with the skin.

5. The line of sealing protective clothes according to claims 1, 2 and 4, characterised in that the inflatable elastic tubular elements (20) are engaged with the annular opening (21) through which the clothes are put on, and associated to non elastic positioning surface belts (26), said elastic tubular elements (20) being provided with a plug-in inflation valve of the type adopted for children's small life-rings and/or dinghies, so as not to constitute surface disturbances and not to get in touch with the skin, said valves being or being not provided with nonreturn internal membranes, and said elastic tubular bodies (20) being inflatable by hand pumps (24) or compressed air cylinders (25) or like means, through the valves (23).

6. The line of sealing protective clothes according to claims 1, 2, 4 and 5, characterised in that the inflated tubular elements (20) are maintained in sealing contact against the body parts of the patients by means of non elastic external belts (26) which cause their fitting to the skin surfaces; said external belts operating the positioning by forming a ring non-extensible towards the outside that causes the tubular element to expand, during its inflation, only towards the inside, adhering to the skin and following its possible curves; and said belts being provided which closing means such as buckles, hooks, press-studs, pressure sensible adhesives (28) of the Velcro type, or the like.

7. The line of sealing protective clothes according to claims 1, 2, 4-6, characterised in that for clothes of the type of trousers or pants with long (6), medium (6') or short (6") legs and for clothes of the type of body-belts or part thereof, the tubular elements (20) comprise a back pocket (27) filled with silicone or other fluid plastic products, facing the inside and aligneable with the back zone corresponding to the spine.

8. The line of sealing protective clothes according to claims 1-7, characterised in that the clothes made from elastic, flexible and insulating material are tailored in a traditional maker or ring-like rolled up around openings (3A), (5A), (7A), through which said clothes are put on.

9. The line of sealing protective clothes according to claims 1-8, characterised in that the clothes made from elastic, flexible and insulating material, suitable for the protection of well defined parts of the human body, are realised with forms like pantyhose (1); more or less extended leggings (3), (3'); short or long socks (4); trousers or pants with long (6), medium (6') or short (6") legs; body-belts of parts thereof, protection of the shoulders and/or part of the back which are put on through the arms and wherein the parts covering the shoulders may also be developed along the arms, up to the hands, possibly comprised; gloves (9) with armlets of various length, wrist- (10), elbow- (10") and armpit- (10") long; armlets (11) designed for the protection of the elbow, arm or forearm, and the like; said configurations being always provided with annular strips (2), (2') and/or inflatable sealing elastic tubular elements (20), located in correspondence of the openings (21) through which the clothes are put on.

10. The line of sealing protective clothes according to claims 1-9, characterised in that the clothes made from elastic, flexible and insulating material may be so configured as to adhere to the body parts, or to have an ample configuration, not to adhere to the body, except for the strips (2), (2') and/or the sealing inflatable elastic elements (20), located in correspondence of the openings (21) through which said clothes are put on, which maintain their characteristic sealing adherence with the corresponding parts of the body against which they engage.

11. The line of sealing protective clothes according to claims 1-10, characterised in that the clothes made from elastic, flexible and insulating material may be of the re-usable type or the disposable type.
